# EUROPEAN PATENT APPLICATION

(11) **EP 4 238 961 A1**
(43) Date of publication of application: **06.09.2023**
(21) Application number: 21886364.5
(22) Date of filing: 29.10.2021
(51) Int. Cl.: C07D 209/08, A61K 31/422, A61P 35/00, B01J 31/24, C07B 61/00, C07D 413/06

(54) **METHOD OF PRODUCING 3-METHYL-4-HALO-INDOLE DERIVATIVE**

(30) Priority: 30.10.2020 JP 2020182237
(71) Applicant: Daiichi Sankyo Company, Limited, Tokyo 103-8426 (JP)
(72) Inventor: SAKURAI Natsuki, Tokyo 103-8426 (JP); SAKAMOTO Tatsuhiro, Tokyo 103-8426 (JP); OGURA Tomokazu, Tokyo 103-8426 (JP)
(74) Representative: Fish & Richardson P.C.
(86) International application number: PCT/JP2021/039946
(87) International publication number: WO 2022/092247

(57) **Abstract**

The present inventors found a novel method for reducing formyl groups with the use of a Lewis acid and a hydride reducing agent in a production method for a 3-methyl-4-haloindole derivative. The present inventors also found that demethylation and/or dehalogenation of the derivative are suppressed by this method, making post-treatment easier or the derivative obtained at a high yield.

## Description

### Technical Field

The present invention relates to a novel method for producing 3-methyl-4-haloindole derivatives and, in particular, a production method including a novel method for reducing formyl haloindole derivatives using Lewis acids and hydride reducing agents.

### Background Art

3-Methyl-4-haloindole derivatives are known to be useful as pharmaceuticals or raw materials in their production, and are known to be useful in the treatment of tumors (Patent Document 1).

Patent Document 1 discloses various types of 3-methyl-4-haloindole derivatives and production methods therefor. For example, in a 3-methyl-4-haloindole derivative production method disclosed in this document, 3-formyl-4-bromo-7-fluoroindole is used as a raw material, and a 3-methyl indole derivative is obtained using a reduction reaction with sodium bis (2-methoxyethoxy) aluminum hydride (Patent Document 1, Reference Example E-14).

However, the yield from this method is modest, resins derived from sodium bis (2-methoxyethoxy) aluminum hydride are produced in post-treatment, making the operation difficult to handle, and the reagents used are expensive.

### Citation List

### Patent Literature

Patent Document 1: WO 2016/052697 A1

### Summary of Invention

### Technical Problem

It is an object of the present invention to provide a method for producing 3-methyl-4-haloindole derivatives, which is a novel, industrially useful production method including a novel reduction method for formyl haloindole derivatives.

### Solution to Problem

As a result of extensive research conducted to achieve the object stated above, the present inventors found that by reducing the formyl groups with the use of a Lewis acid and a hydride reducing agent in the production of a 3-methyl-4-haloindole derivative, the derivative could be obtained at a high yield, or demethylation and/or dehalogenation of the derivative could be suppressed and post-treatment made easier. They also found that polymorphs of mono (2-methylpropan-2-ammonium)=(2E)-3-(1-{[5-(2-fluoropropan-2-yl)-3-(2,4,6-trichlorophenyl)-1,2-oxazol-4-yl]carbonyl}-3-methyl-1H-indol-4-yl) propa-2-enoate could be produced using an intermediate obtained using this method. The present invention is a product of these findings.

The present invention relates to (1) to (17) below.
(1) A production method comprising a step of reducing a compound represented by Formula (I) or a pharmaceutically acceptable salt thereof using a Lewis acid having one type of metal ion selected from a Li ion, a Mg ion, a Ca ion, a Sc ion, a Bi ion, a Ti ion, an Fe ion, a Cu ion, a Ce ion, a La ion, and a Yb ion, and a hydride reducing agent 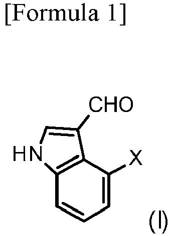
   to obtain a compound represented by Formula (II) or a pharmaceutically acceptable salt thereof 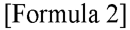 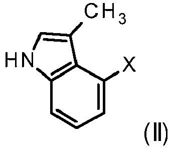
   [wherein X represents a halogen atom].
(2) A production method according to (1), wherein X is a bromine atom.
(3) A production method according to (1) or (2), wherein the Lewis acid is a Lewis acid containing one type of metal ion selected from a Mg ion, a Sc ion, a Bi ion, a Ti ion, a La ion, and a Yb ion.
(4) A production method according to (1) or (2), wherein the Lewis acid is a Lewis acid containing a Ti ion.
(5) A production method according to (1) or (2), wherein the Lewis acid is Ti(OR)₄, in which R is a C₁-C₄ alkyl group.)
(6) A production method according to any one of (1) to (5), wherein R is an ethyl group, an isopropyl group, or a butyl group.
(7) A production method comprising a step of reacting a compound represented by Formula (II) produced using a production method according to any one of (1) to (6) with tert-butyl acrylate using a palladium catalyst to obtain a compound represented by Formula (III) or a pharmaceutically acceptable salt thereof. 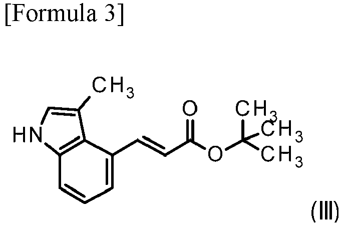
(8) A production method comprising a step of condensing a compound represented by Formula (III) produced using a production method according to (7) with a compound represented by Formula (IV) 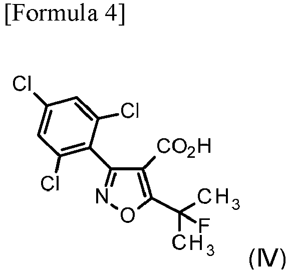 to obtain a compound represented by Formula (V). 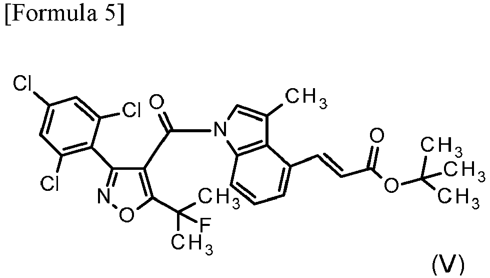
(9) A production method comprising a step of hydrolyzing a compound represented by Formula (V) produced using a production method according to (8) to obtain a compound represented by Formula (VI) or a pharmaceutically acceptable salt thereof. 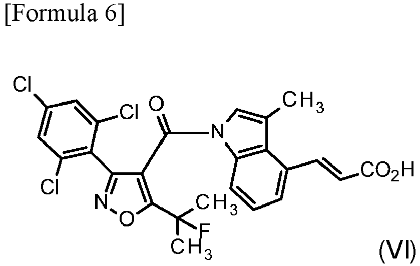
(10) A method for producing a compound represented by Formula (VI) using a compound represented by Formula (II) produced using a production method according to any one of (1) to (6) as an intermediate.
(11) A method for producing a tert-butyl amine salt of a compound represented by Formula (VI), comprising the steps of salifying a compound represented by Formula (VI) produced using a production method according to (9) or (10), and
   crystallizing the product in a mixed solution of acetone and 2-propanol.
(12) A tBA1 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 5.81 ± 0.2, 10.31 ± 0.2, 11.09 ± 0.2, 11.54 ± 0.2, 15.56 ± 0.2, 16.19 ± 0.2, 19.24 ± 0.2, 23.16 ± 0.2, 25.80 ± 0.2, and 26.28 ± 0.2 in a powder X-ray diffraction pattern obtained by irradiation with Kα rays (λ = 1.54 angstroms) of copper.
(13) A tBA2 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.23 ± 0.2, 6.35 ± 0.2, 9.51 ± 0.2, 12.64 ± 0.2, 15.79 ± 0.2, 16.67 ± 0.2, 18.99 ± 0.2, 20.62 ± 0.2, 25.42 ± 0.2, 28.06 ± 0.2, and 28.42 ± 0.2 in a powder X-ray diffraction pattern obtained by irradiation with Kα rays (λ = 1.54 angstroms) of copper.
(14) A P1 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.15 ± 0.2, 14.92 ± 0.2, 15.55 ± 0.2, 18.70 ± 0.2, 20.40 ± 0.2, 23.20 ± 0.2, 25.13 ± 0.2, 26.13 ± 0.2, 27.86 ± 0.2, and 28.81 ± 0.2 in a powder X-ray diffraction pattern obtained by irradiation with Kα rays (λ = 1.54 angstroms) of copper.
(15) AP2 type crystal of mono (2-methylpropan-2-ammonium)=(2E)-3-(1-{ [5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.04 ± 0.2, 9.08 ± 0.2, 18.23 ± 0.2, 24.38 ± 0.2, 24.66 ± 0.2, and 27.18 ± 0.2 in a powder X-ray diffraction pattern obtained by irradiation with Kα rays (λ = 1.54 angstroms) of copper.
(16) A P3 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.10 ± 0.2, 6.23 ± 0.2, 9.39 ± 0.2, 12.55 ± 0.2, 15.71 ± 0.2, 18.15 ± 0.2, 18.91 ± 0.2, 25.32 ± 0.2, 27.10 ± 0.2, and 27.94 ± 0.2 in a powder X-ray diffraction pattern obtained by irradiation with Kα rays (λ = 1.54 angstroms) of copper.
(17) A pharmaceutical composition containing a crystal according to (12) to a crystal according to (16) as an active ingredient.

### Effects of Invention

The present invention is able provide a novel, industrially useful production method for 3-methyl-4-halo-indole derivatives. In particular, the present invention can provide a high-yield production method including a novel reduction method for formyl groups that suppresses dehalogenation and/or demethylation and that makes post-treatment easier by using a Lewis acid and a hydride reducing agent. Polymorphs of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate can be produced using this production method.

### Brief Description of Drawings

[Fig. 1]

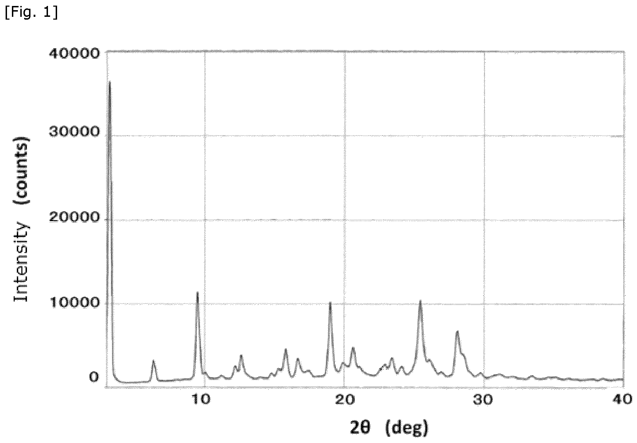

   Fig. 1 shows the powder X-ray diffraction pattern of crystals of the compound produced in Example 7-1. The vertical axis in the figure indicates the diffraction intensity as a relative line intensity, and the horizontal axis indicates the diffraction angle 2θ.
[Fig. 2]

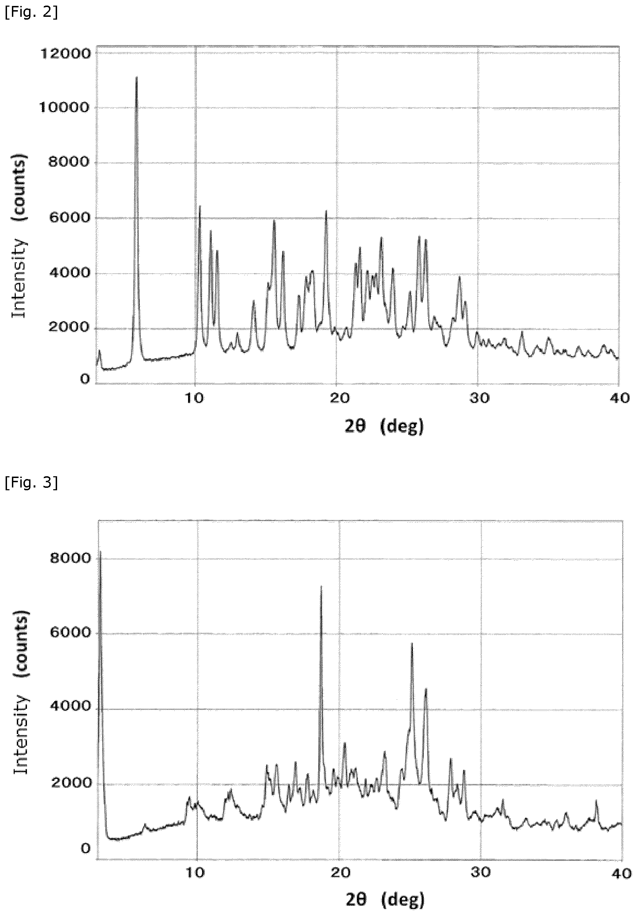

   Fig. 2 shows the powder X-ray diffraction pattern of crystals of the compound produced in Example 8-1. The vertical axis in the figure indicates the diffraction intensity as a relative line intensity, and the horizontal axis indicates the diffraction angle 2θ.
[Fig. 3]
   Fig. 3 shows the powder X-ray diffraction pattern of crystals of the compound produced in Example 8-2. The vertical axis in the figure indicates the diffraction intensity as a relative line intensity, and the horizontal axis indicates the diffraction angle 2θ.
[Fig. 4]

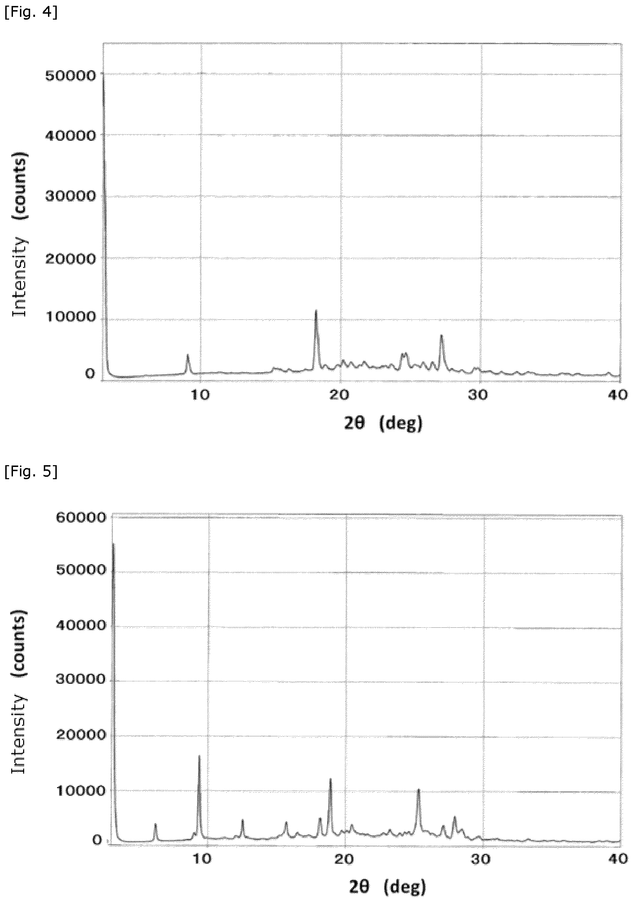

   Fig. 4 shows the powder X-ray diffraction pattern of crystals of the compound produced in Example 8-3. The vertical axis in the figure indicates the diffraction intensity as a relative line intensity, and the horizontal axis indicates the diffraction angle 2θ.
[Fig. 5]
   Fig. 5 shows the powder X-ray diffraction pattern of crystals of the compound produced in Example 8-4. The vertical axis in the figure indicates the diffraction intensity as a relative line intensity, and the horizontal axis indicates the diffraction angle 2θ.

### Description of Embodiments

### (Production Method of the Present Invention)

The present invention relates to the following production method.

A production method comprising a step of reducing a compound represented by Formula (I) or a pharmaceutically acceptable salt thereof using a Lewis acid and a hydride reducing agent 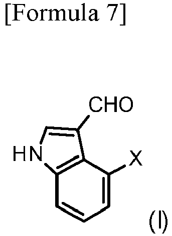
to obtain a compound represented by Formula (II) or a pharmaceutically acceptable salt thereof 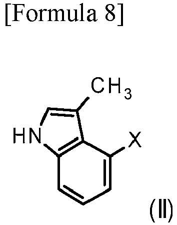
[wherein X represents a halogen atom].

This method, as shown in the examples below, is a novel and industrially useful production method (a production method that obtains a high yield of a derivative, that suppresses demethylation and/or dehalogenation of the derivative, and that makes post-treatment easier) that includes a novel formyl-haloindole derivative reduction technique.

In the present invention, "Lewis acid" means a substance containing metal ions that can accept an electron pair. Examples of metal ions in Lewis acids include a Li ion, Mg ion, Ca ion, Al ion, Sc ion, In ion, Bi ion, B ion, Ti ion, Fe ion, Co ion, Cu ion, Zn ion, Ce ion, La ions, and Yb ion. From the standpoint of easily obtaining a high yield of a compound represented by Formula (II) or a pharmaceutically acceptable salt, a Li ion, Mg ion, Ca ion, Sc ion, Bi ion, Ti ion, Fe ion, Cu ion, Ce ion, La ion, or Yb ion is preferred, a Mg ion, Ca ion, Sc ion, Bi ion, Ti ion, Cu ion, La ion, or Yb ion is more preferred, a Mg ion, Sc ion, Bi ion, Ti ion, La ion, or Yb ion is even more preferred, and a Ti ion is especially preferred. A "high yield" in the present invention means that the HPLC area ratio shown in the examples below is preferably 75 area% or higher.

The valences of the metal ions contained in a Lewis acid of the present invention include Li⁺, Mg²⁺, Ca²⁺, Al³⁺, Sc³⁺, In³⁺, Bi³⁺, B³⁺, Ti⁴⁺, Fe²⁺, Fe³⁺, Co²⁺, Co³⁺, Cu⁺, Cu²⁺, Zn²⁺, Ce³⁺, Ce⁴⁺, La³⁺, and Yb³⁺. The preferred valences of each are Li⁺, Mg²⁺, Ca²⁺, Al³⁺, Sc³⁺, In³⁺, Bi³⁺, B³⁺, Ti⁴⁺ , Fe³⁺, Co²⁺, Cu⁺, Zn²⁺, Ce³⁺, La³⁺, and Yb³⁺.

Lewis acids containing Li ions include LiCl, LiBr, LiI, and LiOTf. Lewis acids containing Mg ions include MgCl₂, MgBr₂, and MgI₂. MgCl₂ is preferred. Lewis acids containing Ca ions include CaCl₂, CaBr₂, and CaI₂. CaCl₂ is preferred. Lewis acids containing Al ions include A1C1₃, AlBr₃, and AlI₃. AlCl₃ is preferred. An example of a Lewis acid containing Sc ions is Sc(OTf)₃. Lewis acids containing In ions include InCl₃, InBr₃, and InI₃. Lewis acids containing Bi ions include BiCl₃, BiBr₃, and Bi(OTf)₃. BiCl₃ is preferred. An example of a Lewis acid containing B ions is BF₃·Et₂O. Lewis acids containing Ti ions include, for example, TiCl4 and Ti(OR)₄ (such as Ti(OEt)4, Ti(OiPr)4 and Ti(OBu)4). Ti(OR)₄ is preferred, and Ti(OEt)4, Ti(OiPr)4 or Ti(OBu)4 is more preferred. In Here, R represents a C₁-C₄ alkyl group described later. Lewis acids containing Fe ions include FeCl₂, FeBr₂, FeI₂, FeCl₃, FeBr₃, and FeI₃. FeCl₃ is preferred. Lewis acids containing Co ions include CoCl₂, CoBr₂, and CoI₂. CoCl₂ is preferred. Lewis acids containing Cu ions include CuCl, CuBr, CuI, CuCl₂, CuBr₂, and CuI₂. CuI is preferred. Lewis acids containing Zn ions include ZnCl₂, ZnBr₂, Zn(Otf)₂, and ZnO. Lewis acids containing Ce ions include CeCl₃ and CeCl₄. An example of a Lewis acid containing La ions is La(OTf)₃. An example of a Lewis acid containing Yb ions is Yb(OTf)₃.

In the present invention, preferred Lewis acids are Lewis acids containing Mg ions, Lewis acids containing Ca ions, Lewis acids containing Sc ions, Lewis acids containing Bi ions, Lewis acids containing Ti ions, Lewis acids containing Cu ions, Lewis acids containing La ions, and Lewis acids containing Yb ions. MgCl₂, MgBr₂, MgI₂, CaCl₂, Sc(OTf)₃, BiCl₃, Ti(OEt)₄, Ti(OiPr)₄, Ti(OBu)₄, CuI, La(OTF)₃, and Yb(OTf)₃ are more preferred. Ti(OEt)₄, Ti(OiPr)₄, and Ti(OBu)₄ are especially preferred.

In the production of 3-methyl-4-halo-indole derivatives, it can be difficult to remove the impurity (imp.2) shown in the examples section below by purification. Therefore, in the present invention, from the standpoint of easily suppressing the generation of imp.2, that is, the demethylation of a compound represented by Formula (II) or a pharmaceutically acceptable salt thereof, the Lewis acid in the present invention is preferably a Lewis acid containing Mg ions, Lewis acid containing Al ions, Lewis acid containing Sc ions, Lewis acid containing In ions, Lewis acid containing Bi ions, Lewis acid containing B ions, Lewis acid containing Ti ions, Lewis acid containing Fe ions, Lewis acid containing Co ions, Lewis acid containing Cu ions, Lewis acid containing La ions, or a Lewis acid containing Yb ions. From the standpoint of easily obtaining a high yield of a compound represented by Formula (II) or a pharmaceutically acceptable salt thereof while also readily suppressing the formation of imp.2, the Lewis acid in the present invention is more preferably a Lewis acid containing Mg ions, a Lewis acid containing Sc ions, a Lewis acid containing Bi ions, a Lewis acid containing Ti ions, a Lewis acid containing Fe ions, and a Lewis acid containing Cu ions, a Lewis acid containing La ions, or a Lewis acid containing Yb ions, and even more preferably MgCl₂, MgBr₂, MgI₂, Sc(OTf)₃, BiCl₃, Ti(OEt)₄, Ti(OiPr)₄, Ti(OBu)₄, FeCl₃, CuI, La(OTf)₃, or Yb(OTf)₃. In the present invention, "suppressing the formation of imp.2" means that the HPLC area ratio of imp.2 shown in the examples section below is preferably 3 area% or less, and more preferably 2 area% or less.

Similarly, in the production of 3-methyl-4-halo-indole derivatives, from the standpoint of production of imp. 1, that is, readily suppressing dehalogenation of a compound represented by Formula (II) or a pharmaceutically acceptable salt thereof, the Lewis acid in the present invention is preferably a Lewis acid containing Bi ions, a Lewis acid containing Cu ions, a Lewis acid containing Ti ions, a Lewis acid containing Li ions, a Lewis acid containing Zn ions, and a Lewis acid containing Ca ions, a Lewis acid containing Yb ions, a Lewis acid containing Sc ions, or a Lewis acid containing Mg ions, more preferably a Lewis acid containing Bi ions, a Lewis acid containing Cu ions, a Lewis acid containing Ti ions, a Lewis acid containing Li ions, or a Lewis acid containing Zn ions, and even more preferably BiCl₃, Bi(OTf)₃, BiBr₃, CuI, TiCl₄, LiI, LiBr, LiCl, LiOTf, or ZnO. In the present invention, "suppressing the formation of imp. 1" means that the HPLC area ratio of imp. 1 shown in the examples section below is preferably 10 area% or less, and more preferably 5 area% or less.

In the present invention, a "C₁-C₄ alkyl group" is a linear or branched alkyl group having from 1 to 4 carbon atoms. Examples include a methyl group, ethyl group, propyl group, isopropyl group, butyl group, isobutyl group, s-butyl group, and t-butyl group. An ethyl group, isopropyl group, or butyl group is preferred. An ethyl group is especially preferred.

In the present invention, "hydride reducing agents" that can be used include sodium borohydride, lithium borohydride, calcium borohydride, sodium triacetoxyborohydride, and lithium triethylborohydride. Sodium borohydride is preferred.

Solvents used in the present invention should be inert in each reaction. In a reduction reaction using a hydride reducing agent, for example, N-methyl-2-pyrrolidone, N,N-dimethylacetamide, isopropanol, tetrahydrofuran, or any mixture of these solvents can be used. Use of N-methyl-2-pyrrolidone, isopropanol, tetrahydrofuran, or any mixture of these solvents is preferred.

In the present invention, a compound represented by Formula (I) or a pharmaceutically acceptable salt thereof is reduced using a Lewis acid mentioned above, hydride reducing agent, and solvent to obtain a compound represented by Formula (II) or a pharmaceutical product thereof. The specific reaction conditions are described in greater detail in the examples section below (see especially Examples 1-1 to 1-3). In this way, a person skilled in the art can produce a compound represented by Formula (II) or a pharmaceutically acceptable salt thereof while using the proper reaction conditions. However, the reaction conditions of the present invention should not be interpreted as being limited to those listed in the examples section.

The present invention is also a production method comprising a step of reacting a compound represented by Formula (II) produced using the production method described above with tert-butyl acrylate using a palladium catalyst to obtain a compound represented by Formula (III) 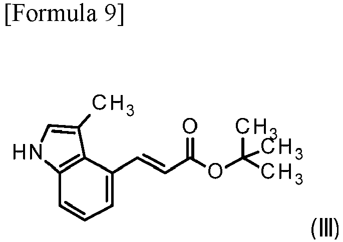 or a pharmaceutically acceptable salt thereof.

The present invention is also a production method comprising a step of condensing a compound represented by Formula (III) produced using the production method described above with a compound represented by Formula (IV) 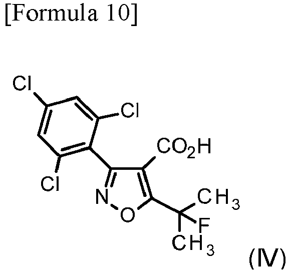 to obtain a compound represented by Formula (V).

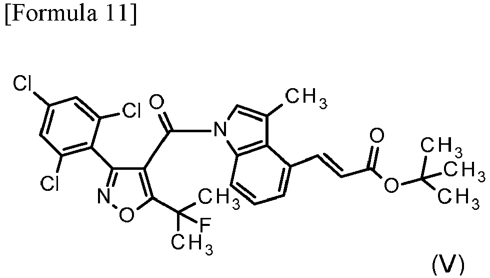

The present invention is also a production method comprising a step of hydrolyzing a compound represented by Formula (V) produced using the production method described above to obtain a compound represented by Formula (VI) 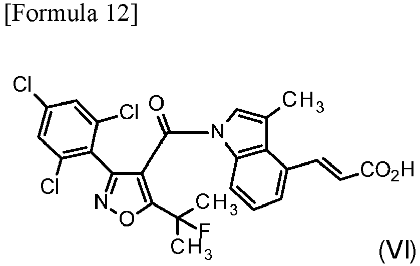 or a pharmaceutically acceptable salt thereof.

The present invention also relates to a method for producing a compound represented by Formula (VI) using a compound represented by Formula (II) produced using the production method described above as an intermediate.

The present invention also relates to a method for producing a tert-butyl amine salt of a compound represented by Formula (VI), comprising the steps of salifying a compound represented by Formula (VI) produced by the production method described above using tert-butylamine, and
crystallizing the product in a mixed solution of acetone and 2-propanol.

The method for producing a tert-butylamine salt of a compound represented by Formula (VI) from a compound represented by Formula (II) or a pharmaceutically acceptable salt thereof is also described in detail in the examples section below (see especially Examples 4-1 to 8-4). This method is also described in detail in Patent Document 1. In this way, compound represented by formula (III) or a pharmaceutically acceptable salt thereof, a compound represented by formula (V), and a compound represented by formula (VI) or a pharmaceutically acceptable salt thereof can each be prepared while using the proper reaction conditions. However, the methods for producing these compounds in the present invention should not be interpreted as being limited to those described below.

In the present invention, the functional groups in the compounds may be protected by a suitable protecting group. Examples of functional groups include hydroxyl groups, carboxy groups, and amino groups. For types of protecting groups and the conditions for introducing and removing these protecting groups, see Protective Groups in Organic Synthesis (T. W. Green and P.G.M. Wuts, John Wiley & Sons, Inc., New York, 2006).

### (Compounds of the Present Invention)

The following is a detailed description of compounds represented by Formulas (I) to (VI), or pharmaceutically acceptable salts thereof, which are the reactants, intermediates, and products in the described production methods described above.

In the compounds represented by Formula (I) and the compounds represented by Formula (II) in the present invention, "X" is a halogen atom, and examples include a fluorine atom, a chlorine atom, a bromine atom, and an iodine atom, preferably a bromine atom.

In the present invention, compounds represented by Formula (III) and pharmaceutically acceptable salts thereof, compounds represented by Formula (V), and compounds represented by Formula (VI) and pharmaceutically acceptable salts thereof include geometrical isomers.

In the present invention, a "pharmaceutically acceptable salt" refers to a salt that does not have significant toxicity and can be used as a pharmaceutical composition.

In the present invention, a compound represented by Formula (I), a compound represented by Formula (II), and a compound represented by Formula (III) can be converted into a salt by reacting it with an acid. Examples include hydrogen halides such as hydrofluorides, hydrochlorides, hydrobromide, and hydroiodides; inorganic acid salts such as nitrates, perchlorates, sulfates, and phosphates; C₁-C₆ alkyl sulfonates such as methane sulfonates, trifluoromethane sulfonates, and ethane sulfonates; aryl sulfonates such as benzene sulfonates and p-toluene sulfonates; organic acid salts such as acetates, malates, fumarates, succinates, citrates, ascorbates, tartarates, oxalates, and adipates; and amino acid salts such as glycine salt, lysine salt, arginine salt, ornithine salt, glutamic acid salts, and aspartic acid salts.

In the present invention, compounds represented by Formula (IV) and compounds represented Formula (VI) can be converted into a salt by reacting them with a base. Examples include alkali metal salts such as sodium salts, potassium salts, and lithium salts; alkaline metal salts such as calcium salts and magnesium salts; metal salts such as aluminum salts and iron salts; inorganic salts such as ammonium salts; and organic salts such as t-butylamine salts, t-octylamine salts, dibenzylamine salts, morpholinate salts, glucosamine salts, phenylglycine alkyl ester salts, ethylenediamine salts, N-methylglucamine salts, guanidine salts, diethylamine salts, triethylamine salts, dicyclohexylamine salts, N, N'-dibenzylethylenediamine salts, chloroprocaine salts, procaine salts, diethanol amine salts, N-benzyl phenethylamine salts, piperazine salts, tetramethyl ammonium salts, and tris (hydroxymethyl) aminomethane salts.

In the present invention, compounds represented by Formula (I) and pharmaceutically acceptable salts thereof, compounds represented by Formula (II) and pharmaceutically acceptable salts thereof, compounds represented by Formula (III) and pharmaceutically acceptable salts thereof, compounds represented by Formula (IV) and pharmaceutically acceptable salts thereof, compounds represented by Formula (V), and compounds represented by Formula (VI) and pharmaceutically acceptable salts thereof may take up water molecules and become hydrates by being allowed to stand in the air or by recrystallization. These hydrates are also incorporated into the present invention.

In the present invention, compounds represented by Formula (I) and pharmaceutically acceptable salts thereof, compounds represented by Formula (II) and pharmaceutically acceptable salts thereof, compounds represented by Formula (III) and pharmaceutically acceptable salts thereof, compounds represented by Formula (IV) and pharmaceutically acceptable salts thereof, compounds represented by Formula (V), and compounds represented by Formula (VI) and pharmaceutically acceptable salts thereof may absorb a solvent and become solvates by being allowed to stand in a solvent or by recrystallization. These solvates are also incorporated into the present invention.

The tert-butylamine salts of compounds represented by Formula (VI) of the present invention also include the following crystals.

A tBA1 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks (for example, 3, 5, 6, 7, 8, 9, or 10 peaks)) at a diffraction angle (2Θ) selected from 5.81 ± 0.2, 10.31 ± 0.2, 11.09 ± 0.2, 11.54 ± 0.2, 15.56 ± 0.2, 16.19 ± 0.2, 19.24 ± 0.2, 23.16 ± 0.2, 25.80 ± 0.2, and 26.28 ± 0.2 in a powder X-ray diffraction pattern obtained by irradiation with Kα rays (λ = 1.54 angstroms) of copper.

A tBA2 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks (for example, 3, 5, 6, 7, 8, 9, 10, or 11 peaks) at a diffraction angle (2Θ) selected from 3.23 ± 0.2, 6.35 ± 0.2, 9.51 ± 0.2, 12.64 ± 0.2, 15.79 ± 0.2, 16.67 ± 0.2, 18.99 ± 0.2, 20.62 ± 0.2, 25.42 ± 0.2, 28.06 ± 0.2, and 28.42 ± 0.2 in a powder X-ray diffraction pattern obtained by irradiation with Kα rays (λ = 1.54 angstroms) of copper.

A P1 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks (for example, 3, 5, 6, 7, 8, 9, or 10 peaks) at a diffraction angle (2Θ) selected from 3.15 ± 0.2, 14.92 ± 0.2, 15.55 ± 0.2, 18.70 ± 0.2, 20.40 ± 0.2, 23.20 ± 0.2, 25.13 ± 0.2, 26.13 ± 0.2, 27.86 ± 0.2, and 28.81 ± 0.2 in a powder X-ray diffraction pattern obtained by irradiation with Kα rays (λ = 1.54 angstroms) of copper.

A P2 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks (for example, 3, 5, or 6 peaks) at a diffraction angle (2θ) selected from 3.04 ± 0.2, 9.08 ± 0.2, 18.23 ± 0.2, 24.38 ± 0.2, 24.66 ± 0.2, and 27.18 ± 0.2 in a powder X-ray diffraction pattern obtained by irradiation with Kα rays (λ = 1.54 angstroms) of copper.

A P3 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks (for example, 3, 5, 6, 7, 8, 9, or 10 peaks) at a diffraction angle (2Θ) selected from 3.10 ± 0.2, 6.23 ± 0.2, 9.39 ± 0.2, 12.55 ± 0.2, 15.71 ± 0.2, 18.15 ± 0.2, 18.91 ± 0.2, 25.32 ± 0.2, 27.10 ± 0.2, and 27.94 ± 0.2 in a powder X-ray diffraction pattern obtained by irradiation with Kα rays (λ = 1.54 angstroms) of copper.

A compound represented by Formula (VI) of the present invention or a pharmaceutically acceptable salt thereof can be prepared as a pharmaceutical composition or as a reagent for research purposes (see Patent Document 1).

When preparing a compound of the present invention or a pharmaceutically acceptable salt thereof as a pharmaceutical composition, examples of pharmaceutically acceptable carriers that can be used include, but are not limited to, sterile water or saline, vegetable oils, solvents, bases, emulsifiers, suspending agents, surfactants, stabilizers, flavorants, fragrances, excipients, vehicles, preservatives, binders, diluents, tonicity agents, analgesics, bulking agents, disintegrants, buffering agents, coating agents, lubricants, coloring agents, sweetening agents, thickening agents, flavoring agents, solubilizing agents, and other additives. The compound of the present invention or a pharmaceutically acceptable salt thereof can be made into various forms, such as tablets, powders, granules, capsules and liquids depending on the purpose of the treatment. It can also be administered, for example, in the form of a liposome delivery system. These liposomes can include auxiliary moieties (antibodies, ligands, etc.) that enhance their therapeutically useful properties.

There are no particular restrictions on the target disease of a pharmaceutical composition of the present invention, but preferred examples include brain tumors (including glioma), acute myelogenous leukemia, myelodysplastic syndrome, myeloproliferative tumor, peripheral T-cell lymphoma, chondrosarcoma, osteosarcoma, cholangiocarcinoma, primitive neuroectodermal tumor, B-origin lymphoblastic lymphoma, malignant melanoma, prostate cancer, colon cancer, and thyroid cancer, Ollier's disease, and Maffucci's syndrome. In other words, a pharmaceutical composition of the present invention can be used as an antitumor agent.

There are no particular restrictions on the active ingredient of a pharmaceutical composition of the present invention as long as it is a compound represented by Formula (VI) or a pharmaceutically acceptable salt thereof. However, the active ingredient is preferably a tert-butylamine salt of the compound represented by Formula (VI), more preferably, a crystal selected from the group consisting of a tBA1-type crystal, tBA2-type crystal, P1-type crystal, P2-type crystal, or P3-type crystal of a tert-butylamine salt, and even more preferably a tBA1-type crystal, tBA2-type crystal, P1-type crystal, P2-type crystal, or P3-type crystal.

### Examples

The following is a more detailed description of the present invention with reference to examples, but the scope of the present invention is not limited to these examples. The abbreviations used in the examples have the following meanings.
mg: milligram, g: gram, mL: milliliter, L: liter, MHz: megahertz. NMP: N-methylpyrrolidone

In the examples below, the nuclear magnetic resonance (hereinafter, ¹H NMR: 500 MHz) spectrum is described in terms of chemical shift δ value (ppm) using tetramethylsilane as the reference substance. In the splitting pattern, s indicates a singlet, d indicates a doublet, t indicates a triplet, q indicates a quadruplet, m indicates a multiplet, and br indicates a broad singlet. In the examples below, HPLC 10A (Shimadzu) or Acquity UPLC H-Class (Waters) was used in the liquid chromatography.

The following device and measurement conditions were used in the powder X-ray diffraction measurements performed on the examples.
Model: Rigaku MiniFlex 600
Sample size: Appropriate amount
X-ray generating conditions: 40 kV, 15 mA
Wavelength: 1.54 Å (copper Kα line)
Measurement temperature: Room temperature
Scanning speed: 10°/min
Scanning range: 3 to 40°
Sampling width: 0.02°

In general, the position of a peak in an X-ray diffraction spectrum can be expected to vary by about ± 0.2° 2θ. In other words, when the difference between the 2θ values of two peaks being compared is within the range of about ± 0.2° 2θ, then both peaks are considered to be the same peak.

### (Reference Example 1-1) Production of Methyl=5-(2-fluoropropan-2-yl)-3-(2,4,6-trichlorophenyl)-1,2-oxazol-4-carboxylate

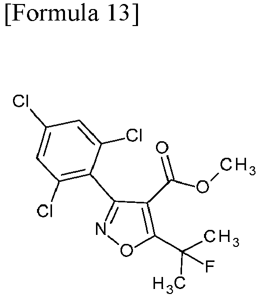

In a nitrogen atmosphere, acetonitrile (120 mL) and 1,1-carbonyldiimidazole (64.19 g, 0.3959 mol) were added to reaction vessel 1. After stirring at room temperature, a solution of 2-fluoro-2-methylpropanoic acid (40 g) in acetonitrile (40 mL) was added dropwise over 30 minutes, and the contents were stirred for another 30 minutes. Next, in a nitrogen atmosphere, acetonitrile (200 mL) and monomethylpotassium malonate (70.66 g, 0.4524 mol) were added to reaction vessel 2, and magnesium chloride (28.72 g, 0.3016 mol) was added while keeping the interior temperature in a range from 10 to 40°C. Afterwards, the solution in reaction vessel 1 was added dropwise to reaction vessel 2 at 50°C over 40 minutes, and the contents were washed with acetonitrile (40 mL). After cooling to room temperature, a mixed solution of concentrated hydrochloric acid (100 mL) and water (280 mL) was added dropwise over 15 minutes, stirred for 5 minutes, allowed to stand for 5 minutes, and then separated to obtain an acetonitrile solution of methyl=4-fluoro-4-methyl-3-oxopentanoate.

Separately, in a nitrogen atmosphere, dimethylformamide (70 mL) and N-hydroxy-1- (2,4,6-trichlorophenyl) methaneimine (10 g) were added to a reaction vessel. After stirring at room temperature, a solution of trichloroisocyanuric acid (3.73 g, 0.0160 mol) in ethyl acetate (25.0 mL) was added dropwise while keeping the interior temperature in a range from 10 to 40°C, and then the contents were washed with ethyl acetate (5.0 mL). After stirring at room temperature for 30 minutes, a solution of sodium chloride (5.0 g) in water (45.0 mL) was added, and the contents were stirred for 5 minutes, allowed to stand for 5 minutes, and separated. The aqueous layer was then discarded. Next, water (50 mL) was added, and the contents were stirred for 5 minutes, allowed to stand for 5 minutes, and separated. The aqueous layer was then discarded. Finally, a solution of sodium chloride (10.0 g) in water (40.0 mL) was added, and the contents were stirred for 5 minutes, allowed to stand for 5 minutes, and separated to obtain an ethyl acetate solution of 2,4,6-trichloro-N-hydroxybenzene-1-carboxyimidoyl chloride.

In a nitrogen atmosphere, an acetonitrile solution of methyl=4-fluoro-4-methyl-3-oxopentanoate (equivalent to 0.068 mol) was added to the reaction vessel, and the interior temperature was adjusted to 15°C. After adding a methanol solution of 28 (w/w)% sodium methoxide (12.0 g, 0.0623 mol) at 15°C, a solution of 2,4,6-trichloro-N-hydroxybenzene-1-carboxyimidoyl chloride (equivalent to 0.0445 mol) in acetate was added dropwise over 1 hour, and the contents were washed with ethyl acetate (5 mL). After the dropwise addition, the contents were stirred at an interior temperature of 15°C for 1 hour, water (25 mL) was added at room temperature, and the pH was adjusted from 7 to 9 with 2 mol/L hydrochloric acid. After stirring for 15 minutes and allowing the contents to stand for 5 minutes, the liquid was separated and the aqueous layer was discarded. A solution of sodium chloride (10.0 g) in water (40.0 mL) was added, and the contents were stirred for 15 minutes, allowed to stand for 5 minutes, and separated. The aqueous layer was then discarded. The resulting organic layer was concentrated under reduced pressure to 50 mL or lower at 50°C or less. Next, 2-propanol (200 mL) was added, and the contents were concentrated again under reduced pressure to 50 mL or lower at 50°C or less. The volume of the solution was adjusted to 60 mL with 2-propanol to obtain 55.79 g of 2-propanol solution. A portion of the solution was extracted, the interior temperature was adjusted to 5°C, and the contents were stirred. After confirmation of crystallization, the contents were stirred for another 30 minutes. Water (10.6 mL) was added dropwise over 2 hours, and the contents were stirred for 30 minutes. The resulting suspension was filtered, and the crystals were washed with a mixture of cooled 2-propanol (4 mL) and water (4 mL) and then dried under reduced pressure at 40° C overnight to obtain the target compound (2.55 g, 0.00696 mol).

¹HNMR (500 MHz, DMSO-d₆) δ2.14 (d, J = 20 Hz, 6H) 3.90 (s, 3H), 8.18 (s, 2H); ESI MS m/z 366 ([M+1]+).

### (Reference Example 1-2) Production of Methyl 5-(2-fluoropropan-2-yl)-3-(2,4,6-trichlorophenyl)-1,2-oxazole-4-carboxylate

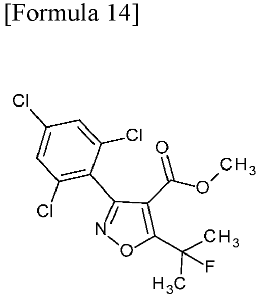

In a nitrogen atmosphere, acetonitrile (348 g) and methyl 2-fluoro-2-propionate (145 g) were added to a reaction vessel, and the contents were stirred at 0°C. While keeping the internal temperature below 10°C, 1,1-carbonyldiimidazole (244.1 g) was added dropwise over one hour in five portions. The inlet was washed with acetonitrile (20 g) and the contents were stirred for an another hour. Afterward, the temperature in the reaction vessel was adjusted to 25°C in a nitrogen atmosphere, acetonitrile (435g) and potassium monomethylmalonate (235g) were added to the reaction vessel 2, and magnesium chloride (143.5 g) was added in four portions while keeping the internal temperature below 40°C or less. The inlet was washed with acetonitrile (145g) and the contents were stirred at 25°C for one hour. The solution obtained in reaction vessel 1 was added dropwise to reaction vessel 2 at 30°C over one hour, and washed with acetonitrile (20 g). Stirring was continued at 30°C for three hours and then the contents were allowed to cool to 20°C. A mixture of concentrated hydrochloric acid (428 g) and ordinary water (362.5) was added dropwise over 1 hour, and the contents were stirred for 30 minutes. After allowing the product to stand for 30 minutes, liquid separation was performed, 10% aqueous sodium chloride solution (725 g) was added, the contents stirred for 30 minutes, allowed to stand still for another 30 minutes, and then subjected to liquid separation again. The same operation was repeated three times to obtain an acetonitrile solution of methyl=4-fluoro-4-methyl-3-oxopentanoate.

In a nitrogen atmosphere, dimethylacetamide (900 g) and N-hydroxyl-1-(2,4,6-trichlorophenyl)methyleneimine (215 g) were added to a reaction vessel, and the inlet was washed with dimethylacetamide (37 g). The contents were stirred at 50°C until clear, N-chlorosuccinimide (126 g) was added in 21 portions over three hours while keeping the internal temperature below 55°C, and the contents were stirred at 50°C for another hour. After stirring the contents at 25°C for 30 minutes, a solution of methyl tert-butyl ether (1450 g) and sodium chloride (100 g) in normal water (900 mL) was added, the contents were stirred for 30 minutes and allowed to stand for 30 minutes, and the aqueous layer was separated out and discarded. The saline washing and liquid separation operations were repeated twice, and a methyl tert-butyl ether solution of 2,4,6-trichloro-N-hydroxybenzene-1-carboximide chloride was obtained.

In a nitrogen atmosphere, the acetonitrile solution of methyl=4-fluoro-4-methyl-3-oxopentanoate was added to the reaction vessel, industrial water (188.5 mL) was added, and the internal temperature was adjusted to 5°C. A methanol solution of sodium methoxide (223.3 g) was added at 30°C or less within two hours to adjust the pH to 12-14. A methyl tert-butyl ether solution of 2,4,6-trichloro-N-hydroxybenzene-1-carboximide chloride was added dropwise at 20°C for two hours, and the contents were washed with acetonitrile (145 g). After the dropwise addition, the contents were stirred at an internal temperature of 20°C for one hour, and a solution of ethyl acetate (1450 g) and sodium chloride (145 g) in industrial water (1305 g) was added at room temperature. The contents were stirred for 30 minutes, allowed to stand for 30 minutes, and the aqueous layer was separated out and discarded. The same saline washing procedure was repeated twice. The resulting organic layer was concentrated under reduced pressure to 400 mL or less at 45°C or less. Methanol (725 g) was added, and the contents were concentrated under reduced pressure at 45°C or lower until the volume became 400 mL or lower. Methanol (725 g) was added a second time and the contents were concentrated under reduced pressure at 45°C to 400 mL or less. After adding methanol (478.5 g), the contents were stirred at 40°C for 20 minutes, and industrial water (101.5 mL) was added dropwise to the reaction vessel over one hour. After adding seed crystals (0.435 mg, seed crystals produced according to Reference Example 1-1), the contents were stirred for two hours. Ordinary water (350 mL) was added dropwise over three hours, and the contents were stirred for two hours. The contents were cooled to 20°C over four hours and then stirred for another four hours. The resulting suspension was filtered, and the crystals were washed with a mixture of cooled methanol (232 g) and ordinary water (145 g) and dried under reduced pressure at 40°C for 24 hours to obtain the desired compound (295.0 g, 0.80 mol, yield 88.1%). The ¹H-NMR values of the resulting target compound were compared with those shown in Reference Example 1-1 to confirm that they were the same compound.

### (Reference Example 2-1) Production of 5-(2-Fluoropropan-2-yl)-3-(2,4,6-trichlorophenyl)-1,2-oxazol-4-carboxylic acid

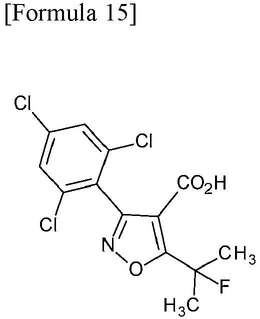

In a nitrogen atmosphere, methanol (30 mL) was added to a reaction vessel, and the compound (5 g) obtained in Reference Example 1 was added while stirring. A 40 (w/w)% tetrabutylammonium hydroxide aqueous solution (9.73 g, 0.0150 mol) was added, and the contents were stirred at 72°C for 3 hours. The temperature was adjusted to 40°C, 2 mol/L hydrochloric acid (5.1 mL) was added, and the contents were stirred for 1 hour after confirmation of crystallization. 2 mol/L hydrochloric acid (2.7 mL) was added dropwise over 10 minutes, and the contents were stirred for 10 minutes. Water (20 mL) was added dropwise over 10 minutes, and the contents were stirred for 10 minutes, cooled to room temperature, and then stirred for 30 minutes. The resulting suspension was filtered, and the crystals were washed with a mixture of methanol (7.5 mL) and water (7.5 mL) and dried under reduced pressure at 40°C overnight to obtain the target compound (4.44 g , 0.0126 mol, yield 92.3%).

¹HNMR (500 MHz, DMSO-d₆) δ1.89 (d, J = 20 Hz, 6H), 7.89 (s, 2H); ESI MS m/z 350 ([M-1]-).

### (Reference Example 2-2) Production of 5-(2-Fluoropropan-2-yl)-3-(2,4,6-trichlorophenyl)-1,2-oxazole-4-carboxylic acid

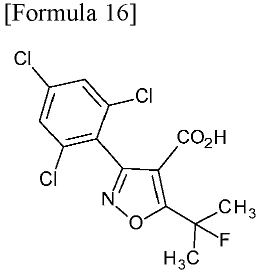

In a nitrogen atmosphere, methanol (440.0 g) was added and stirred in a reaction vessel, the compound (5 g) obtained in Reference Example 1-2 was added, and 40 (w/w)% tetrabutylammonium was added. An aqueous hydroxide solution (195.0 g) was then added, and the contents were stirred at 45°C for 20 hours. The temperature was adjusted to 40°C and 2mol/L hydrochloric acid (145.0g) was added. After crystallization was confirmed, the contents were stirred for another hour. Next, 2 mol/L hydrochloric acid (75.0 g) was added dropwise over one hour, and the contents were stirred for 2 hours. Then, ordinary water (600 g) was added dropwise over one hour, and the contents were stirred for one hour. After cooling to 20°C, the contents were stirred for another hour. The resulting suspension was filtered, and the crystals were washed with a mixture of methanol (120 g) and ordinary water (150 g) and dried under reduced pressure at 40°C for 20 hours to obtain the target compound (yield 95.3%). The ¹H-NMR values of the resulting target compound were compared with those shown in Reference Example 2-1 to confirm that they were the same compound.

### (Example 1-1) Production of 4-Bromo-3-methyl-1H-indole

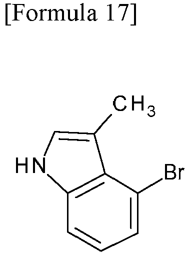

In a nitrogen atmosphere, N-Methylpyrrolidone (70 mL), tetraisopropyl ortho-titanate (19.03 g, 0.06695 mol) and sodium borohydride (6.75 g, 0.178 mol) were added to the reaction vessel, and the contents were stirred at room temperature for 1 hour. After cooling to 0°C, the raw material (20 g) dissolved in N-methylpyrrolidone (20 mL) was added dropwise. After raising the temperature to 80°C, the contents were stirred at 80°C for 2 hours. The contents were cooled again to 0°C, acetone (39.5 mL) was added dropwise, and the contents stirred at 0°C for 13.5 hours. Then, lactic acid (37.5 g) was added dropwise at 0°C, the contents were stirred at 0°C for 1 hour, and the temperature was raised to room temperature. After adding water (60 mL) and sodium chloride (6 g), ethyl acetate (300 mL) was added, the contents were stirred at room temperature, and then the aqueous layer was removed. After adding water (60 mL) and sodium chloride (12 g) to the organic layer, the contents were stirred at room temperature and then the aqueous layer was removed. After adding water (60 mL) and sodium hydrogen carbonate (4.8 g) to the organic layer, the contents were stirred at room temperature, and the aqueous layer was removed to obtain an ethyl acetate and N-methylpyrrolidone solution of the target compound (16.72 g, 0.07959 mol, yield 89.2%).

¹HNMR (500 MHz, CDCl₃) δ2.54 (s, 3H), 6.95-6.98 (m, 2H), 7.23 (d, J = 7.8 Hz, 2H), 7.88(brs, 1H).

### (Example 1-2) Production of 4-Bromo-3-methyl-1H-indole

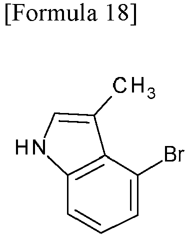

In a nitrogen atmosphere, tetrahydrofuran (2.08 kg), starter material 4-bromo-1H-indole-3-formaldehyde (0.80 kg, 3.571 mol), and isopropanol (3.76 kg) were added to reaction vessel 1, and the contents were stirred for one hour at 25°C. Purified water was replenished until the solution KF (water in system) was 0.5%, the contents were cooled to 5°C, and sodium borohydride (144.0 g, 1.06 eq) was added in portions to prepare a mixture of the intermediate (4-bromo-1H-indol-3-yl)methanol in tetrahydrofuran and isopropanol, which was then stored below 5°C. At the same time, tetrahydrofuran (2.08 kg) and isopropanol (3.76 kg) were added to reaction vessel 2, and then sodium borohydride (152.0 g, 1.13 eq) and CaCl₂ (400.0 g, 1.1 eq) were added at 25°C. The solution was stirred at 25°C for 0.5 hour, then warmed to 60°C. The reaction solution of the intermediate (4-bromo-1H-indol-3-yl)methanol reaction was then added dropwise and reacted for 36 hours at 60°C. After completion of the reaction, the temperature of reaction vessel 2 was lowered to 25°C, toluene (7.04 kg (8.8 equivalents relative to the starting material)) was added, the temperature of the reaction vessel was adjusted to 5°C, 14.91 kg of aqueous AcOH (0.51 kg of AcOH solution and 14.40 kg of water) was added to quench the excess sodium borohydride, and the temperature was adjusted 25°C and stirring performed for one hour while keeping the quench temperature below 30°C. After allowing the contents to stand for 0.5 hours, the aqueous layer was discarded. Purified water (8 kg) was added to the organic layer, the contents were stirred at 25°C for one hour, and the aqueous layer was discarded. Then, 4.20 kg of sodium hydrogen carbonate aqueous solution (0.20 kg of sodium hydrogen carbonate dissolved in 4.00 kg of water) was added to the organic layer. After stirring for one hour at room temperature, the aqueous layer was removed, the organic layer was concentrated to 2.4 L, toluene (2.00 kg) was added, the contents were concentrated to 2.4 L, and a toluene solution of the target compound was obtained.

### (Example 2-1) Study of Various Lewis Acids 1

In the reaction performed to obtain compound (2) from compound (1), Lewis acid screening was performed under the same reaction conditions as in Example 1-1, and the changes in the amounts of the various compounds changed were studied from the HPLC area ratio. The results are shown in Table 1. In the following tables, "imp." refers to impurities.

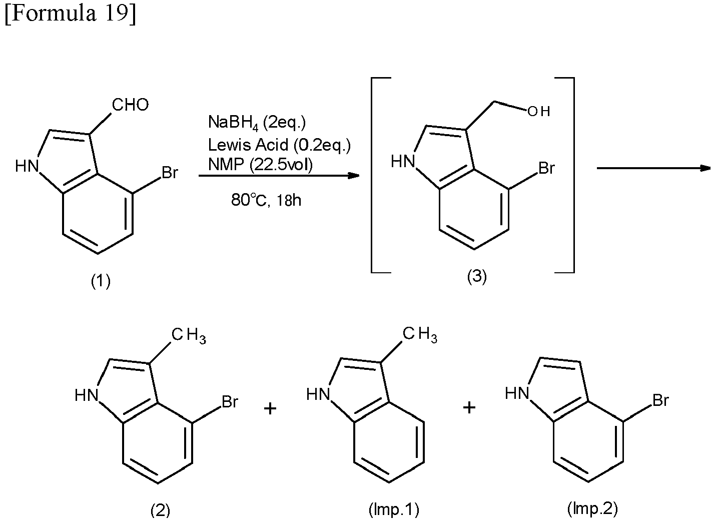

**[Table 1]**

| No. | Lewis Acid | HPLC (225 nm, area%) | | | | |
|---|---|---|---|---|---|---|
| | | 3 | 2 | Imp.1 | Imp.2 | 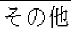 imp. |
| 1 | LiCl | 7.37 | 75.76 | 3.53 | 9.22 | 4.12 |
| 2 | LiEr | 7.2 | 75.15 | 3.42 | 9.46 | 4.47 |
| 3 | LiOTf | 7.21 | 76.39 | 3.58 | 8.8 | 4.02 |
| 4 | MgCl₂ | 2.55 | 85.12 | 8.2 | 1.13 | 3 |
| 5 | MgBr₂ | 2.62 | 84.93 | 7.45 | 1.76 | 3.24 |
| 6 | MgI₂ | 2.27 | 85.93 | 8.04 | 0.8 | 2.96 |
| 7 | CaCl₂ | 3.44 | 84.61 | 5.42 | 3.62 | 2.91 |
| 8 | AlCl₃ | 10.22 | 73.74 | 11.73 | - | 4.31 |
| 9 | S_{C}(OTf)₃ | 3.76 | 86.01 | 7.23 | 0.13 | 2.87 |
| 10 | InBr₃ | 9.57 | 62.5 | 19.45 | - | 8.48 |
| 11 | BiCl₃ | 7.5 | 88.35 | 0.7 | - | 3.45 |
| 12 | BF₃Et₂O | 27.39 | 58.39 | 10.37 | - | 3.85 |
| 13 | TiCl₄ | 9.92 | 78.94 | 2.89 | - | 8.25 |
| 14 | Ti(OiPr)₄ | 3.59 | 86.26 | 7.09 | - | 3.06 |
| 15 | Lil | 6.93 | 76.56 | 3.26 | 9.34 | 3.91 |
| 16 | FeCl₃ | 7.73 | 74.74 | 15.2 | 0.25 | 2.08 |
| 17 | CoBr₂ | - | 0.74 | 93.14 | - | 6.12 |
| 18 | CuI | 13.42 | 80.36 | 1.24 | 0.88 | 4.1 |
| 19 | ZnCl₂, | 23.48 | 67.54 | 7.64 | - | 1.34 |
| 20 | ZnBr₂ | 22.27 | 68.09 | 8.1 | - | 1.54 |
| 21 | ZnO | 9.25 | 72.99 | 3.58 | 9.65 | 4.53 |
| 22 | Zn(OTf)₂ | 23.37 | 68.12 | 7.42 | - | 1.09 |
| 23 | InCl₃ | 6.95 | 58.22 | 30.35 | - | 4.48 |
| 24 | CeCl₃ | 5.64 | 79.94 | 3.41 | 6.97 | 4.04 |
| 25 | La(OTf)₃ | 2.18 | 85.76 | 10.63 | - | 1.43 |
| 26 | Yb(OTf)₃ | 2.66 | 87.15 | 6.32 | 0.51 | 3.36 |
| 27 | Bi(OTf)₃ | 3.91 | 91.83 | 1.83 | 0.74 | 1.69 |
| 28 | BiBr₃ | 6.88 | 86.32 | 3.56 | - | 3.24 |
| 29 | InI₃ | 5.59 | 69.75 | 23.3 | - | 1.36 |
| 30 | Ti(OEt)₄ | 0.83 | 92.13 | 5.63 | - | 1.41 |

### HPLC conditions

Detection: 225 nm
Column: YMC Triart C8 (4.6 mm ID x 150 mm, 3 µm)
Column temperature: 40°C
Mobile phase: A: 10 mM ammonium acetate aqueous solution, B: acetonitrile

### Gradient conditions:

**[Table 2]**

| | | | | | | |
|---|---|---|---|---|---|---|
| Time (min) | 0 | 3 | 13 | 15 | 15,1 | 20 |
| B sIn. conc. (%) | 40 | 40 | 90 | 90 | 40 | I 40 |

Flow rate: 1.0 mL/min
Injection volume: 1 µL
Sample solution: Acetonitrile/water (8:2)

### (Example 2-2) Study of Various Lewis Acids 2

The Lewis acid equivalents of some of the studied Lewis acids in Table 1 were studied under the same reaction conditions as in Example 1. The results are shown in Table 3. The HPLC conditions are the same as those in Example 2-1. Also, the reaction system is preferably in a solution state rather than a slurry state because it is a more uniform reaction state.

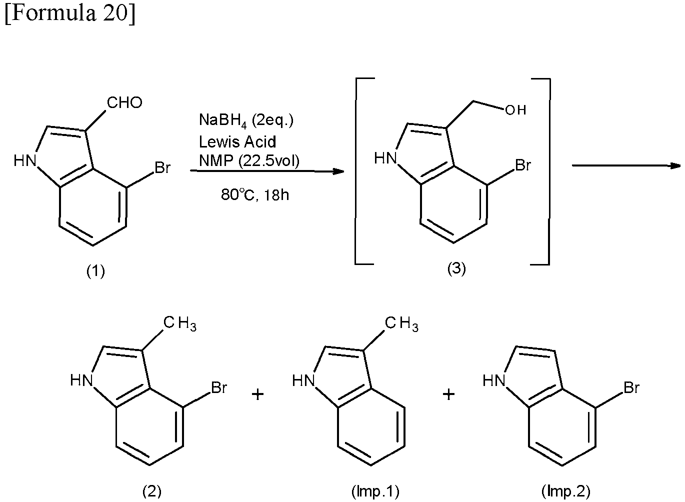

**[Table 3]**

| Lewis acid | Equiv | HPLC (225 nm, area%) | | | | | remarks |
|---|---|---|---|---|---|---|---|
| | | 3 | 2 | Imp.1 | Imp.2 | Other imp. | |
| MgCl₂ | 0.5 | 1 | 86.85 | 9.5 | | 2.65 | Slurry |
| | 0.75 | 0.93 | 84.62 | 12.58 | | 1.87 | Slurry |
| | 1 | 1.66 | 83.47 | 11.29 | - | 3.58 | Slurry |
| | 1.5 | 1.34 | 90.95 | 6.5 | - | 1.21 | Slurry |
| BiCl₃ | 0.5 | 10.32 | 77.2 | 0.63 | 0.66 | 11.19 | Slurry |
| | 0.75 | 0.32 | 90.96 | 0.1 | 0.97 | 7.65 | Slurry |
| | 1 | 0.04 | 95.2 | - | 0.68 | 4.08 | Slurry |
| | 1.5 | 0.11 | 81.24 | - | - | 18.65 | Slurry |
| Ti(OEt)₄ | 0.5 | - | 97,22 | 2.41 | - | 0.37 | Solution |
| | 0.75 | 0.22 | 96,61 | 2.88 | - | 0.29 | Solution |
| | | - | 97.91 | 1.79 | - | 0.3 | Solution |
| | 1.5 | - | 98.96 | 0.91 | - | 0.13 | Solution |

### (Example 3) Study of Various Lewis Acids 3

Some titanium alkoxides were examined under the same reaction conditions as in Example 1-1. The results are shown in Table 4. No. 1-3 in the table were all solutions.

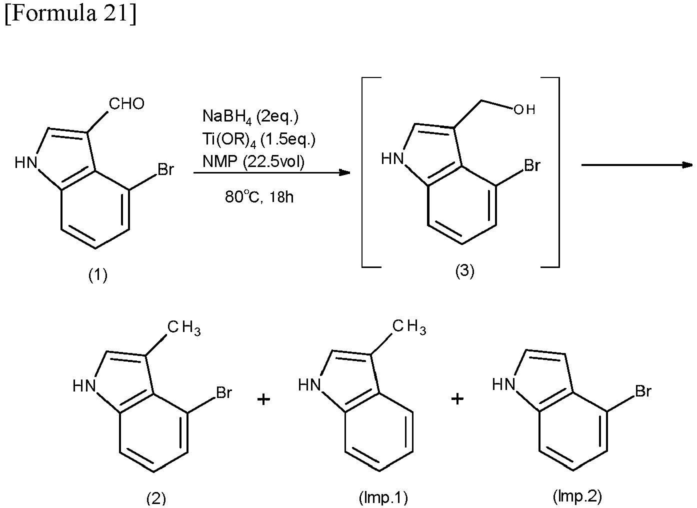

**[Table 4]**

| No. | Ti(OR)₄ | HPLC (225 nm, area%) | | | | |
|---|---|---|---|---|---|---|
| | | 3 | 2 | Imp.1 | Imp. 2 | Other imp. |
| 1 | Ti(OEt)₄ | - | 99.4 | 0.06 | 0.03 | 0.51 |
| 2 | Ti(OiPr)₄ | 0.07 | 99.22 | 0.05 | - | 0.66 |
| 3 | Ti(OnBu)₄ | 0.05 | 99.03 | 0.11 | - | 0.81 |

### (Example 4-1) Production of tert-Butyl=(2E)-3-(3-methyl-1H-indol-4-yl) prop-2-enoate

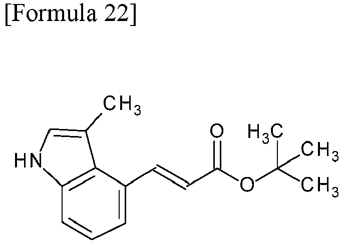

In a nitrogen atmosphere, acetonitrile (25 mL), a N-methylpyrrolidone solution of the compound (5 g) obtained in Example 1-1, palladium acetate (0.053 g, 0.00024 mol), tris (o-tolyl) phosphine (0.145 g, 0.000476 mol), triethylamine (2.89 g, 0.0286 mol), and tert-butyl acrylate (3.66 g, 0.0286 mol) were added to a reaction vessel, and the contents were stirred at 80°C for 3 hours. After cooling to 50°C, water (12.5 mL) was added dropwise, and the contents were cooled to room temperature. Seed crystals (5 mg) of the target compound were added, and the contents were stirred at room temperature for 1 hour. Then, water (37.5 mL) was added dropwise over 1 hour, and the contents were stirred at room temperature for 1 hour. The resulting suspension was filtered, and the crystals were washed with a mixture of acetonitrile (10 mL) and water (15 mL) and then dried under reduced pressure at 40°C overnight to obtain the target compound (5.56 g, 0.0216 mol, yield 90.8%).

¹HNMR (500 MHz, DMSO-d₆) δ1.50 (s, 9H), 2.46 (s, 3H), 3.33 (s, 1H), 6.42 (d, J = 16.0 Hz, 1H), 7.08 (t, J = 8.1 Hz, 1H), 7.23 (s, 1H), 7.39 (d, J = 8.1 Hz, 1H), 7.44 (d, J = 7.5 Hz, 1H), 8.39 (d, J = 16.1 Hz, 1H), 10.98 (brs, 1H).

Seed crystals were obtained by purifying the compound using column chromatography and then allowing the purified compound to stand.

### (Example 4-2) Production of tert-Butyl (2E)-3-(3-methyl-1H-indol-4-yl)prop-2-enoate

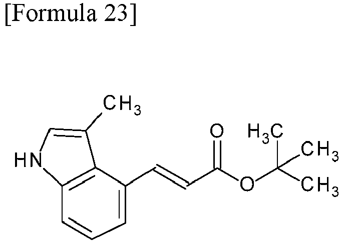

In a nitrogen atmosphere, N,N-dimethylformamide (3.04 kg), a toluene solution of the compound (0.80 kg) obtained in Example 1-2, palladium acetate (8.0 g, 0.0357 mol), tris (p-tolyl)phosphine (28.0 g, 0.107 mol), diisopropyltriethylamine (0.64 kg, 4.95 mol), and tert-butyl acrylate (0.64 kg, 4.93 mol) were added to a reaction vessel, and the contents were stirred at 100°C for 12 hours. After cooling to 50° C., purified water (0.12 kg) was added dropwise and the contents were concentrated to 5.6 L so that the toluene content was 2% or less. Afterward, the system was cooled to 25°C, acetonitrile (2.80 kg) was added, and replenishment continued until the water content of the system reached 10%. Seed crystals (40.0 g) of the target compound were added, and the contents were stirred at 25°C for two hours. Purified water (1.20 kg) was slowly added dropwise over four hours, followed by rapid addition of purified water (5.60 kg) over four hours and stirring at room temperature for another four hours. The resulting suspension was filtered, and the crystals were washed twice with acetonitrile (1.20 kg) and purified water (2.40 kg) and dried under reduced pressure at 40°C for 40 hours to obtain the target compound (0.70 kg, 2.72 mol, yield 76.0%). Seed crystals were obtained by allowing the target compound to remain in a column after purification. The ¹H-NMR values of the resulting target compound were compared with those of the compound in Example 4-1 to confirm that they were the same compound.

### (Example 5-1) Production of tert-butyl=(2E)-3-(1-{ [5-(2-fluoropropan-2-yl)-3-(2,4,6-trichlorophenyl)-1,2-oxazol-4-yl] carbonyl}-3-methyl-1H-indol-4-yl) prop-2-enoate

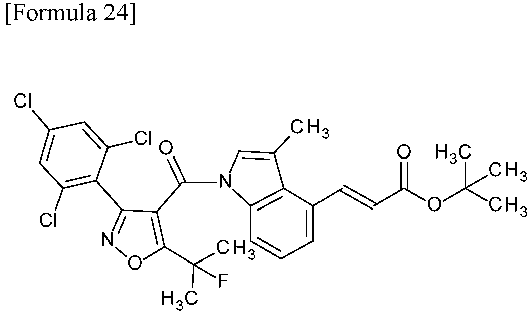

In a nitrogen atmosphere, acetonitrile (100 mL), the compound obtained in Reference Example 2-1 (10 g), and 1,1-carbonyldiimidazole (5.06 g, 0.0284 mol) were added to a reaction vessel, and the contents were stirred at 40°C for 3.5 hours. After cooling to room temperature, the contents were degassed under reduced pressure for 10 minutes, and the compound (8.19 g) obtained in Example 4-1 and diazabicycloundecene (0.86 g, 0.0057 mol) were added. After stirring at room temperature for 2 hours, acetic acid (0.85 g, 0.014 mol) was added. Next, water (43 mL) was added dropwise over 1 hour, and the contents were stirred at room temperature for 15 hours. The resulting suspension was filtered, and the crystals were washed with a mixture of acetonitrile (24 mL) and water (16 mL) and then dried under reduced pressure at 40°C overnight to obtain the target compound (15.94 g, 0.02693 mol, yield 95.0%).

¹HNMR (500 MHz, DMSO-d₆) δ1.49 (s, 9H), 1.83 (d, J = 21.8 Hz, 6H), 2.37 (s, 3H), 6.50 (d, J = 15.5 Hz, 1H), 7.34 (t, J = 8.0 Hz, 1H), 7.44 (s, 1H), 7.73 (d, J = 7.5 Hz, 1H), 7.84 (s, 2H), 8.24 (d, J = 16.1 Hz, 1H), 8.25 (d, J = 8.0 Hz, 1H).

### (Example 5-2) Production of tert-Butyl=(2E)-3-(1-{[5-(2-fluoropropan-2-yl)-3-(2,4,6-trichlorophenyl)-1,2-oxazol-4-yl]carbonyl}-3-methyl-1H-indol-4-yl)prop-2-enoate

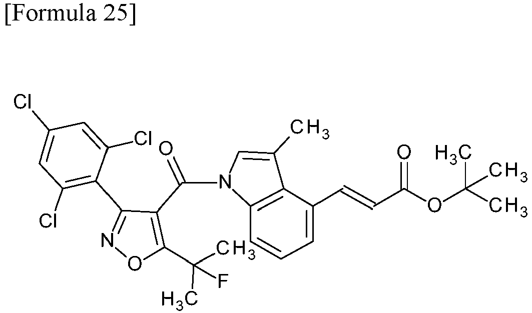

In a nitrogen atmosphere, toluene (36 kg), the compound obtained in Reference Example 2-2 (4.0 kg, 11.3 mol), and 1,1-carbonyldiimidazole (2.28 kg, 14.1 mol) were added to a reaction vessel, and the contents were stirred at 40°C for two hours. After cooling to 25°C, purified water (24 kg) was added, the contents were stirred for one hour and allowed to stand, and the aqueous layer was removed. The organic layer was concentrated to 20 L, and the compound obtained in Example 4-2 (3.03 kg, 11.8 mol), acetonitrile (28 kg), and diazabicycloundecene (346g, 2.27mol) were added when the water content of the system fell below 0.3%. After stirring the contents for six hours at 25°C, acetic acid (340 g, 5.66 mol) was added. Afterward, the reaction solution was concentrated to 20 L, isopropanol (32 kg) was added, and the contents were stirred at 25°C for 1.5 hours. The contents were further concentrated to 20L, isopropanol (32 kg) was added, and the contents were concentrated to 30L. Finally, isopropanol (16 kg) was added, and the contents were stirred at 25°C for 0.5 hours, cooled to -5°C over four hours, and stirred for another four hours. The supernatant retention rate was 0.38%. The resulting suspension was filtered, and the crystals were washed with isopropanol (10 kg) and dried under reduced pressure at 40°C for 18 hours to obtain the target compound (yield 92%). The ¹H-NMR values of the resulting target compound were compared with those of the compound in Example 5-1 to confirm that they were the same compound.

### (Example 6-1) Production of (2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoic acid

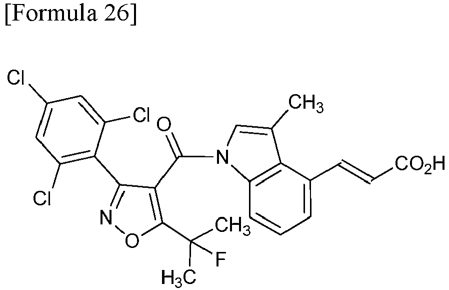

In a nitrogen atmosphere, acetonitrile (100 mL) and the compound obtained in Example 5-1 (10 g) were added to a reaction vessel and stirred at 50°C to completely dissolve the compound. A 12 mol/L hydrochloric acid aqueous solution (4.2 mL) was added, and the contents were stirred for 3 hours and then cooled to room temperature. Then, water (80 mL) was added dropwise over 1 hour, and the contents were stirred at room temperature for 3 hours. The resulting suspension was filtered, and the crystals were washed with a mixture of acetonitrile (22 mL) and water (18 mL) and then dried under reduced pressure at 40°C overnight to obtain the target compound (8.35 g, 0.0558 mol, yield 92.2%).

¹HNMR (500 MHz, DMSO-d₆) δ1.83 (d, J = 21.8 Hz, 6H), 2.37 (s, 3H), 6.50 (d, J = 16.0 Hz, 1H), 7.35 (t, J = 8.0 Hz, 1H), 7.43 (s, 1H), 7.70 (d, J = 7.5 Hz, 1H), 7.84 (s, 2H), 8.25 (d, J = 15.5 Hz, 1H), 8.26 (d, J = 8.6 Hz, 1H).

### (Example 6-2) Production of (2E)-3-(1-{[5-(2-Fluoropropan-2-yl)-3-(2,4,6-trichlorophenyl)-1,2-oxazol-4-yl]carbonyl}- 3-methyl-1H-indol-4-yl)prop-2-enoic acid

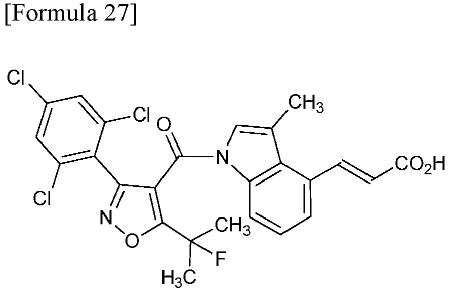

In a nitrogen atmosphere, acetone (25.8 kg) and the compound (3.1 kg) obtained in Example 5-2 were added into a reaction vessel and the contents were stirred at 25°C to dissolve them completely. A 12 mol/L hydrochloric acid aqueous solution (7.4 kg) was added, and the contents were stirred at 40°C for three hours and then cooled to room temperature (20°C). Next, purified water (6.2 kg) was added dropwise over one hour, seed crystals (15.2 g, produced according to Example 6-1) were added, and the contents were stirred at room temperature for one hour. Purified water (12.4 kg) was added dropwise over four hours, and the contents were stirred at 25°C for two hours. The resulting suspension was filtered, and the crystals were washed with a mixture of acetone (6.8 kg) and purified water (9.9 kg) and dried under reduced pressure at 35°C for eight hours to obtain the desired compound (yield: 94.0%). The ¹H-NMR values of the resulting target compound were compared with those of the compound in Example 6-1 to confirm that they were the same compound.

### (Example 7-1) Production of Mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate (also known as (2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate t-butylamine salt)

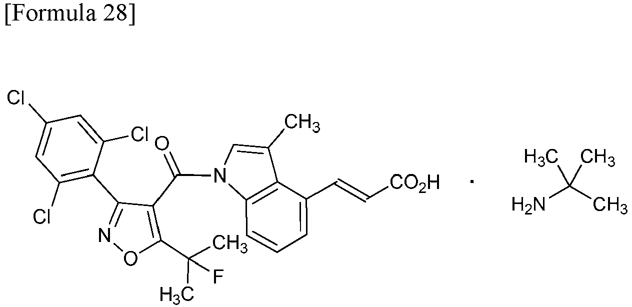

In a nitrogen atmosphere, acetone (255 mL) and 2-propanol (255 mL) were added to the compound (30 g) obtained in Example 6 in a reaction vessel, and the contents were stirred at 30°C for 30 minutes until completely dissolved. Activated carbon (0.3 g) was added and the contents were stirred for 30 minutes. The activated carbon was filtered out, and the activated carbon was washed with a mixture of acetone (45 mL) and 2-propanol (45 mL) to obtain a filtrate. The resulting solution was heated to 40°C, a mixed solution of tert-butylamine (1.2 g), acetone (13 mL), and 2-propanol (13 mL) was added, and the contents were stirred for 30 minutes. After confirming precipitation of seed crystals of the target compound, a mixed solution of tert-butylamine (1.4 g), acetone (15 mL). and 2-propanol (15 mL) was added dropwise over 30 minutes. After stirring the contents for 30 minutes, the temperature was raised to 60°C. A mixture of tert-butylamine (1.6 g), acetone (17 mL), and 2-propanol (17 mL) was then added dropwise over 2 hours. Afterwards, the contents were stirred for 2 hours, cooled to 0°C over 1.5 hours, and then stirred for 3 hours. The resulting suspension was filtered, and the crystals were washed with a mixture of chilled acetone (45 mL) and chilled 2-propanol (45 mL) and then dried under reduced pressure at 40°C overnight to obtain tBA2-type crystals of the target compound (15.5 g). Fig. 1 shows the powder X-ray diffraction pattern, and Table 5 shows the diffraction angle (2θ), the lattice spacing (d value), and the relative intensity in the powder X-ray diffraction spectrum.

¹HNMR (500 MHz, DMSO-d₆) δ1.20 (s, 9H), 1.83 (d, J = 22.4 Hz, 6H), 2.35 (s, 3H), 6.39 (d, J = 15.7 Hz, 1H), 7.30 (t, J = 7.9 Hz, 1H), 7.34 (s, 1H), 7.54 (d, J = 7.9 Hz, 1H), 7.85 (s, 2H), 7.93 (d, J = 15.7 Hz, 1H), 8.17 (d, J = 8.5 Hz, 1H).

**[Table 5]**

| 2θ (°) | d-value (Å) | Relative intensity (%) |
|---|---|---|
| 3.23 | 27.31 | 100 |
| 6.35 | 13.91 | 6 |
| 9.51 | 9.29 | 27 |
| 12.64 | 7 | 7 |
| 15.79 | 5.61 | 9 |
| 16.67 | 5.31 | 6 |
| 18.99 | 4.67 | 22 |
| 20.62 | 4.3 | 8 |
| 25.42 | 3.5 | 22 |
| 28.06 | 3.18 | 11 |
| 28.42 | 3.14 | 6 |

### (Example 7-2) Production of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate

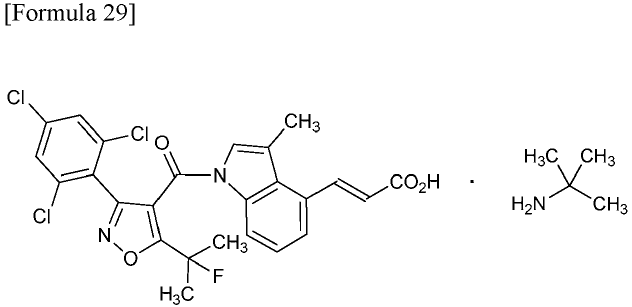

In a nitrogen atmosphere, acetone (32.9 kg) and 2-propanol (32.9 kg) were added to the compound (4.9 kg) obtained in Example 6-2 in a reaction vessel, and the contents were stirred for 30 minutes at 30°C to dissolve them completely. The solution was filtered through ZetaCarbon^{™} (Cuno Filtration S.A.S.), and the ZetaCarbon was washed with a mixture of acetone (5.9 kg) and 2-propanol (5.9 kg) to obtain a solution. The resulting solution was warmed to 40°C, tert-butylamine (200.9 g) was added, and the contents were stirred for one hour. After confirming crystallization of the target compound, tert-butylamine (236.0 g) was added dropwise and stirred with seed crystals (18.5 g, seed crystals produced according to Example 7-1) for one hour while heating to 60°C. tert-Butylamine (169.5 g) was added dropwise and introduced in a line along with acetone (2.5 kg) and 2-propanol (2.5 kg). Afterward, the contents were stirred for three hours, cooled to 0°C over ten hours, and stirred for four hours. The resulting suspension was filtered, and the crystals were washed with a mixture of acetone (5.9 kg) and 2-propanol (5.9 kg). After drying under reduced pressure at 35°C for 20 hours, tBA2-type crystals of the target compound were obtained (yield 87.9%). The ¹H-NMR values of the resulting target compound were compared with those of the compound in Example 7-1 to confirm that they were the same compound.

### (Example 8) Crystal polymorph of mono (2-methylpropan-2-ammonium)=(2E)-3-(1-[5-(2-fluoropropan-2-yl)-3-(2,4,6-trichlorophenyl)-1,2-oxazol-4-yl] carbonyl}-3-methyl-1H-indol-4-yl) propan-2-enoate

The target compound has multiple polymorphs (tBA1, tBA2, P1, P2, P3), and four of these polymorphs are shown in Examples 8-1, 8-2, 8-3, and 8-4, excluding the tBA2 crystals already described in Example 7-1 and Example 7-2.

### (Example 8-1) Production of tBA1-type crystals

Acetone (52 mL) and 2-propanol (52 mL) were added to the compound (10 g) obtained in Example 7-1 and stirred at 30°C for 30 minutes until completely dissolved. Activated carbon (0.1 g) was added over 30 minutes. The activated carbon was then filtered out and the activated carbon was washed with a mixture of acetone (13 mL) and 2-propanol (13 mL) to obtain a filtrate. The resulting solution was heated to 40°C, 2-propanol (80 mL) was added, and a mixture of tert-butylamine (0.2 g), acetone (1.3 mL) and 2-propanol (3 mL) was added. Next, tert-butylamine (1.2 g) was added, and the contents were stirred for 30 minutes and then cooled to 0°C. After stirring for 2 hours, the resulting suspension was washed with a mixture of chilled acetone (15 mL) and chilled 2-propanol (15 mL) and then dried under reduced pressure at 40°C overnight to obtain tBA1 type crystals of the target compound (9.8 g). Fig. 2 shows the powder X-ray diffraction pattern, and Table 6 shows the diffraction angle (2θ), the lattice spacing (d value), and the relative intensity in the powder X-ray diffraction spectrum.

**[Table 6]**

| 2θ (°) | d-value (A) | Relative intensity (%) |
|---|---|---|
| 5.81 | 15.2 | 100 |
| 10.31 | 8.57 | 51 |
| 11.09 | 7.97 | 41 |
| 11.54 | 7.66 | 34 |
| 15.56 | 5.69 | 42 |
| 16.19 | 5.47 | 31 |
| 19.24 | 4.61 | 44 |
| 23.16 | 3.84 | 30 |
| 25.8 | 3.45 | 33, |
| 26.28 | 3.39 | 31 |

### (Example 8-2) Production of P1-type crystals

Acetone (21 mL) and purified water (3 mL) were added to the compound (3 g) obtained in Example 7-1, and the contents were stirred at 40°C for 30 minutes until completely dissolved. Next, tert-butylamine (0.1 g) was added 3 times every 15 minutes, and tert-butylamine (0.1 g) was added after confirming the precipitation of crystals of the target compound. After cooling to 5°C and stirring for 30 minutes, the resulting suspension is filtered, and the crystals were washed with a mixture of cooled acetone (1.5 mL) and cooled purified water (2.6 mL) and then dried overnight under reduced pressure at 40°C to obtain P1-type crystals of the target compound (2.9 g). Fig. 3 shows the powder X-ray diffraction pattern, and Table 7 shows the diffraction angle (2θ), the lattice spacing (d value), and the relative intensity in the powder X-ray diffraction spectrum.

**[Table 7]**

| 2θ (°) | d-value (Å) | Relative intensity (%) |
|---|---|---|
| 3.15 | 28.07 | 100 |
| 14.92 | 5.93 | 17 |
| 15.55 | 5.7 | 16 |
| 18.7 | 4.74 | 86 |
| 20.4 | 4.35 | 18 |
| 23.2 | 3.83 | 16 |
| 25.13 | 3.54 | 52 |
| 26.13 | 3.41 | 47 |
| 27.86 | 3.2 | 21 |
| 28.81 | 3.1 | 18 |

### (Example 8-3) Production of P2-type crystals

Acetone (104 mL) and 2-propanol (104 mL) were added to the compound (20 g) obtained in Example 7-1, and the contents were stirred at 30°C for 30 minutes until completely dissolved. Activated carbon (0.2 g) was added over 30 minutes. The activated carbon was then filtered out and the activated carbon was washed with a mixture of acetone (26 mL) and 2-propanol (26 mL) to obtain a filtrate. The resulting solution was heated to 40°C, 2-propanol (160 mL) was added, and a mixture of tert-butylamine (0.4 g), acetone (2.6 mL) and 2-propanol (6 mL) was added. Next, tert-butylamine (0.9 g) was added. After stirring for 30 minutes, the resulting suspension was filtered, and the crystals were washed with a mixed solution of acetone (30 mL) and 2-propanol (30 mL) and then dried under reduced pressure at 40°C overnight to obtain P2-type crystals of the target compound (17.7 g). Fig. 4 shows the powder X-ray diffraction pattern, and Table 8 shows the diffraction angle (2θ), the lattice spacing (d value), and the relative intensity in the powder X-ray diffraction spectrum.

**[Table 8]**

| 2θ (°) | d-value (Å) | Relative intensity (%) |
|---|---|---|
| 3.04 | 29.01 | 100 |
| 9.08 | 9.73 | 6 |
| 18.23 | 4.86 | 19 |
| 24.38 | 3.65 | 5 |
| 24.66 | 3.61 | 5 |
| 27.18 | 3 .28 | 12 |

### (Example 8-4) Production of P3-type crystals

Acetone (52 mL) and 2-propanol (52 mL) were added to the compound (10 g) obtained in Example 7-1, and the contents were stirred at 30°C for 30 minutes until completely dissolved. Activated carbon (0.1 g) was added over 30 minutes. The activated carbon was then filtered out and the activated carbon was washed with a mixture of acetone (13 mL) and 2-propanol (13 mL) to obtain a filtrate. The resulting filtrate was raised to 60°C, 2-propanol (80 mL) was added, and tert-butylamine (0.5 g) was added. After stirring for 1 hour and confirming precipitation of the target compound, tert-butylamine (0.3 g) was added, the temperature was cooled to 25°C, and the contents were stirred overnight. The resulting suspension was filtered, and the crystals were washed with a mixture of acetone (15 mL) and 2-propanol (15 mL) and then dried under reduced pressure at 40°C overnight to obtain P3-type crystals of the target compound (7.2 g). Fig. 5 shows the powder X-ray diffraction pattern, and Table 9 shows the diffraction angle (2θ), the lattice spacing (d value), and the relative intensity in the powder X-ray diffraction spectrum.

**[Table 9]**

| 2θ (°) | d-value (Å) | Relative intensity (%) |
|---|---|---|
| 3.1 | 28.52 | 100 |
| 6.23 | 14.16 | 6 |
| 9.39 | 9.41 | 30 |
| 12.55 | 7.05 | 7 |
| 15.71 | 5.64 | 6 |
| 18.15 | 4.88 | 7 |
| 18.91 | 4.69 | 21 |
| 25.32 | 3.51 | 17 |
| 27.1 | 3 .29 | 4 |
| 27.94 | 3.19 | 7 |

### Industrial Applicability

As explained above, the present invention is able to obtain a high yield of the derivative in the production of a 3-methyl-4-halo-indole derivative. Demethylation or dehalogenation of the derivative can be suppressed and post-treatment is easier. Derivatives of 3-methyl-4-halo-indole are useful as pharmaceuticals such as antitumor agents or as raw materials in their production, and therefore can be used in the medical field.

## Claims

1. A production method comprising a step of reducing a compound represented by Formula (I) or a pharmaceutically acceptable salt thereof using a Lewis acid having one type of metal ion selected from a Li ion, a Mg ion, a Ca ion, a Sc ion, a Bi ion, a Ti ion, an Fe ion, a Cu ion, a Ce ion, a La ion, and a Yb ion, and a hydride reducing agent 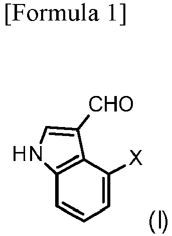 to obtain a compound represented by Formula (II) or a pharmaceutically acceptable salt thereof 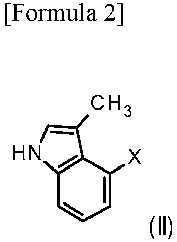 [wherein, X represents a halogen atom].

2. A production method according to claim 1, wherein the Lewis acid is a Lewis acid containing one type of metal ion selected from a Mg ion, a Sc ion, a Bi ion, a Ti ion, a La ion, and a Yb ion.

3. A production method according to claim 1, wherein the Lewis acid is a Lewis acid containing a Ti ion.

4. A production method according to claim 1, wherein the Lewis acid is Ti(OR)₄, in which R is a C₁-C₄ alkyl group.

5. A production method comprising a step of reacting a compound represented by Formula (II) produced using a production method according to any one of claims 1 to 4 with tert-butyl acrylate using a palladium catalyst to obtain a compound represented by Formula (III) or a pharmaceutically acceptable salt thereof.

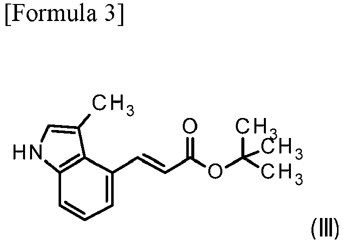

6. A production method comprising a step of condensing a compound represented by Formula (III) produced using a production method according to claim 5 with a compound represented by Formula (IV) 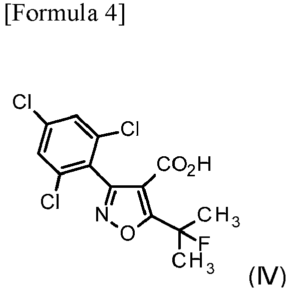 to obtain a compound represented by Formula (V).

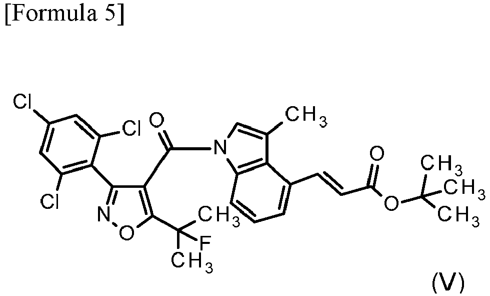

7. A production method comprising a step of hydrolyzing a compound represented by Formula (V) produced using a production method according to claim 6 to obtain a compound represented by Formula (VI) or a pharmaceutically acceptable salt thereof.

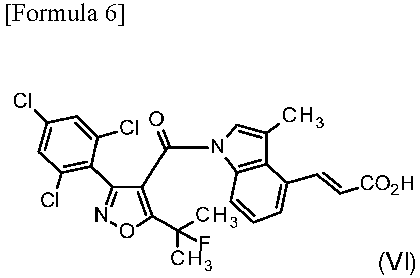

8. A method for producing a tert-butyl amine salt of a compound represented by Formula (VI), comprising the steps of salifying a compound represented by Formula (VI) produced using a production method according to claim 7, and
crystallizing the product in a mixed solution of acetone and 2-propanol.

9. A tBA1 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 5.81 ± 0.2, 10.31 ± 0.2, 11.09 ± 0.2, 11.54 ± 0.2, 15.56 ± 0.2, 16.19 ± 0.2, 19.24 ± 0.2, 23.16 ± 0.2, 25.80 ± 0.2, and 26.28 ± 0.2 in a powder X-ray diffraction pattern obtained by irradiation with Kα rays (λ = 1.54 angstroms) of copper.

10. A tBA2 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.23 ± 0.2, 6.35 ± 0.2, 9.51 ± 0.2, 12.64 ± 0.2, 15.79 ± 0.2, 16.67 ± 0.2, 18.99 ± 0.2, 20.62 ± 0.2, 25.42 ± 0.2, 28.06 ± 0.2, and 28.42 ± 0.2 in a powder X-ray diffraction pattern obtained by irradiation with Kα rays (λ = 1.54 angstroms) of copper.

11. A P1 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.15 ± 0.2, 14.92 ± 0.2, 15.55 ± 0.2, 18.70 ± 0.2, 20.40 ± 0.2, 23.20 ± 0.2, 25.13 ± 0.2, 26.13 ± 0.2, 27.86 ± 0.2, and 28.81 ± 0.2 in a powder X-ray diffraction pattern obtained by irradiation with Kα rays (λ = 1.54 angstroms) of copper.

12. A P2 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.04 ± 0.2, 9.08 ± 0.2, 18.23 ± 0.2, 24.38 ± 0.2, 24.66 ± 0.2, and 27.18 ± 0.2 in a powder X-ray diffraction pattern obtained by irradiation with Kα rays (λ = 1.54 angstroms) of copper.

13. A P3 type crystal of mono (2-methylpropan-2-ammonium)=(2E)-3-(1-{[5-(2-fluoropropan-2-yl)-3-(2,4,6-trichlorophenyl)-1,2-oxazol-4-yl] carbonyl}-3-methyl-1H-indol-4-yl) propa-2-enoate, the crystal having at least three peaks at a diffraction angle (2θ) selected from 3.10 ± 0.2, 6.23 ± 0.2, 9.39 ± 0.2, 12.55 ± 0.2, 15.71 ± 0.2, 18.15 ± 0.2, 18.91 ± 0.2, 25.32 ± 0.2, 27.10 ± 0.2, and 27.94 ± 0.2 in a powder X-ray diffraction pattern obtained by irradiation with Kα rays (λ = 1.54 angstroms) of copper.

14. A pharmaceutical composition containing a crystal according to claim 9 to a crystal according to claim 13 as an active ingredient.
